# EUROPÄISCHE PATENTSCHRIFT

(11) **EP 0 458 110 B1**
(45) Veröffentlichungstag und Bekanntmachung des Hinweises auf die Patenterteilung: **14.12.1994**
(21) Anmeldenummer: 91107255.1
(22) Anmeldetag: 04.05.1991
(51) Int. Cl.: C07D 251/44, C07D 251/50, B01J 19/26

(54) **Verfahren zur kontinuierlichen Umsetzung von Cyanurfluorid mit Aminen.**
Process for continuous reaction of cyanuric fluoride with amines.
Procédé pour la réaction continue de fluorure cyanurique avec des amines.

(30) Priorität: 19.05.1990 DE 4016159
(43) Veröffentlichungstag der Anmeldung: 27.11.1991
(73) Patentinhaber: BAYER AG, 51368 Leverkusen (DE)
(72) Erfinder: Frosch, Hans-Georg, Dr., W-5000 Köln 91 (DE); Hoppe, Manfred, Dr., W-5067 Kürten 2 (DE); Müllers, Wolfgang, Dr., W-5060 Bergisch Gladbach (DE); Stöhr, Frank-Michael, Dr., W-5068 Odenthal (DE)

(56) Entgegenhaltungen:
- EP-A- 0 094 665
- EP-A- 0 172 790
- DE-A- 2 746 109
- DE-B- 2 850 331
- DE-B- 2 850 338
- Ullmanns Encyclopädie der technischen Chemie, 4. Auflage, Band 3, 1973, Seiten 379-385, Verlag Chemie, Weinheim, DE
- Organofluorine Chemicals and Their Industrial Applications, 1979, Seiten 200-206, Verlag R.E. Banks, Horwood Chichester, GB
- Dyes and Pigments, Band 3, 1982, Seiten 160-171

## Beschreibung

Die Erfindung betrifft ein Verfahren zur kontinuierlichen Umsetzung von Cyanurfluorid mit Aminen, wobei Cyanurfluorid und eine wäßrige Aminlösung in einen Reaktor eingeleitet werden.

In DE-A-2 850 331 wird ein Verfahren zur Umsetzung von Aminen mit Cyanurchlorid beschrieben, wobei die Reaktanden durch sich gegenüberliegende Zuleitungen in den Reaktor gelangen.

Aus DE-C2-2 746 109 ist bereits ein Verfahren zur kontinuierlichen Umsetzung von Cyanurfluorid mit Aminobenzolsulfonsäuren oder Aminonaphthalinsulfonsäuren bekannt, wobei als Reaktor ein kontinuierlich durchflossener "Idealkessel" mit vollständiger Rückvermischung der Reaktionsmasse im Reaktor verwendet wird Die dort beschriebene Arbeitsweise ist jedoch nur bedingt anwendbar für die Umsetzung von Cyanurfluorid mit Aminonaphtholsulfonsäuren, da man in diesem Falle erhebliche Mengen an Nebenprodukten erhält.

Aus EP-A2- 0 172 790 ist ferner ein Verfahren zur kontinuierlichen Umsetzung von Cyanurfluorid mit Aminobenzolsulfonsäuren oder Aminonaphthalinsulfonsäuren bekannt, in dem man Cyanurfluorid und eine wäßrige Lösung des Amins gleichzeitig und kontinuierlich in einen ersten Reaktor leitet, dort eine intensive Durchmischung vornimmt, und die Reaktionsmischung anschließend in einen zweiten Reaktor leitet, in dem nur geringe Rückvermischung aber gute radiale Vermischung eintritt, und dort die Umsetzung zu Ende führt.

Diese Arbeitsweise hat jedoch gewisse Nachteile, die darin bestehen, daß man einen zweiten Reaktor benötigt und daß der Anteil der entstehenden Nebenprodukte gegenüber dem in DE-C2-2 746 109 beschriebenen Verfahren zwar vermindert, aber noch immer unbefriedigend hoch ist.

Es besteht die Aufgabe, ein Verfahren und einen Reaktor zu finden, womit die kontinuierliche Umsetzung von Cyanurfluorid mit sulfongruppenhaltigen oder chromophorhaltigen Aminen, insbesondere Aminonaphtholsulfonsäuren zu Aminokondensationsprodukten, d.h. Verbindungen, bei denen 1 Mol Cyanurfluorid mit 1 Mol Amin reagiert, in verbesserter Ausbeute und/oder Reinheit erzielt wird.

Diese Aufgabe wird dadurch gelöst, daß die Edukte gleichzeitig und kontinuierlich mit unterschiedlichen Geschwindigkeiten in den Reaktor eingeleitet werden, daß durch die Differenz der Strömungsgeschwindigkeiten eine intensive Vermischung bewirkt wird, und daß die Reaktion bei rückvermischungsfreier Strömung im wesentlichen in diesem Reaktor abgeschlossen wird.

Überraschenderweise läßt sich nach diesem Verfahren in einer einzigen Reaktionsstufe eine Umsetzung von mindestens 95 % erreichen.

Gemäß einer besonderen Durchführungsform des neuen Verfahrens strömt das Cyanurfluorid mit einer Reynoldszahl von mindestens 10.000, vorzugsweise mindestens 15.000, und die wäßrige Aminlösung mit einer solchen von mindestens 2.500, vorzugsweise mindestens 5.000, in den Reaktor ein, wobei die Differenz der Strömungsgeschwindigkeiten zwischen Cyanurfluoridstrom und Aminlösungsstrom mindestens 20 m/s, vorzugsweise mindestens 40 m/s beträgt.

Diese Maßnahmen gewährleisten eine besonders intensive Vermischung auf kürzestem Wege.

Vorzugsweise wird ein Verhältnis des Reaktorquerschnittes zum Einströmquerschnitt des Cyanurfluoridstromes von 225 bis 40.000, vorzugsweise von 700 bis 12.000, eingehalten.

Durch diese Anpassung des Querschnittsverhältnisses an das Massenstromverhältnis wird die rückströmungsfreie Durchmischung der Reaktionspartner optimiert.

Die Verweilzeit im Reaktor beträgt vorzugsweise maximal 5 s, insbesondere 0,2 bis 2 s.

Es hat sich gezeigt, daß innerhalb dieser Zeit die Umsetzung nahezu vollständig ist.

Gemäß einer weiteren besonderen Durchführungsform des neuen Verfahrens wird die Reaktion bei Temperaturen von 0 bis 50°C, vorzugsweise 0 bis 20°C, durchgeführt.

Besonders geeignet ist das neue Verfahren für die Umsetzung von 2,4,6-Trifluor-s-triazin mit sulfohaltigen Anilinen und Naphthylaminen oder mit sulfohaltigen Aminonaphtholen sowie für die Umsetzung von 2,4,6-Trifluor-s-triazin mit chromophorhaltigen Aminen.

Besonders vorteilhaft ist es, einen rohrförmigen Reaktor zu verwenden, in dem das Cyanurfluorid als axialer Strahl und die Aminlösung als konzentrischer Strahl mit einer eine intensive Vermischung bewirkenden Differenzgeschwindigkeit eingebracht werden, wobei die Reaktion im wesentlichen in diesem Reaktor abgeschlossen wird.

Bei dieser Verfahrensweise findet ein intensiver Stoffaustausch an der Grenzfläche zwischen beiden Strahlen statt.

Überraschenderweise erhält man bei dieser Arbeitsweise auch die Umsetzungprodukte von 1 Mol Aminonaphtholsulfonsäure mit 1 Mol Cyanurfluorid in hoher Ausbeute und Reinheit, Auch für die Reaktion von Aminobenzol- oder Aminonaphthalinsulfonsäuren oder chromophorhaltigen Aminen mit Cyanurfluorid eignet sich das erfindungsgemäße Verfahren in vielen Fällen besser als die bekannten Verfahren.

Das erfindungsgemäße Verfahren kann aber auch mit Ammoniak oder aliphatischen Aminen, z.B. Ethanolamin, Morpholin oder Taurin, durchgeführt werden. Vorzugsweise werden aber sulfogruppenhaltige aromatische Amine und chromophorhaltige Amine eingesetzt, wobei die negativen Ladungen der Sulfogruppen auch durch quartäre, insbesondere aliphatische Ammoniumionen ausgeglichen werden können.

Geeignete sulfogruppenhaltige aromatische Amine sind z.B.:
1-Aminobenzol-2-sulfonsäure, 1-Aminobenzol-3-sulfonsäure, 1-Amino-benzol-4-sulfonsäure, 1-Amino-4-methylbenzol-3-sulfonsäure, 1-Amino-4-methoxybenzol-3-sulfonsäure, 1-Amino-2-methylbenzol-4-sulfonsäure, 1-Amino-3-methylbenzol-4-sulfonsäure, 1-Aminobenzol-3,5-disulfonsäure, 2-Amino-5-sulfobenzoesäure, 1-Aminonaphthalin-4-sulfonsäure, 1-Amino-naphthalin-5-sulfonsäure, 1-Amino-naphthalin-6-sulfonsäure, 2-Aminonaphthalin-5-sulfonsäure, 2-Aminonaphthalin-7-sulfonsäure, 2-Amino-naphthalin-4,8-disulfonsäure, 2-Aminonaphthalin-5,7-disulfonsäure, 1,4-Diaminobenzol-2,5-disulfonsäure, 1,3-Diaminobenzol-4-sulfonsäure, 1,4-Diaminobenzol-2-sulfonsäure, 1,3-Diaminobenzol-4,6-disulfonsäure, 1-Amino-5-hydroxynaphthalin-7-sulfonsäure, 1-Amino-8-hydroxynaphthalin-4-sulfonsäure, 1-Amino-8-hydroxynaphthalin-3-sulfonsäure, 1-Amino-8-hydroxynaphthalin-5-sulfonsäure, 2-Amino-5-hydroxynaphthalin-7-sulfonsäure, 2-Amino-6-hydroxynaphthalin-8-sulfonsäure, 2-Amino-8-hydroxynaphthalin-6-sulfonsäure, 2-Methylamino-5-hydroxynaphthalin-7-sulfonsäure, 2-Ethylamino-5-hydroxynaphthalin-7-sulfonsäure, 2-Methylamino-8-hydroxynaphthalin-6-sulfonsäure, 2-Ethylamino-8-hydroxynaphthalin-6-sulfonsäure, 1-Amino-6-hydroxynaphthalin-3,8-disulfonsäure, 1-Amino-8-hydroxynaphthalin-3,6-disulfonsäure, 1-Amino-8-hydroxynaphthalin-2,4-disulfonsäure, 1-Amino-8-hydroxynapthalin-4,6-disulfonsäure, 1-Amino-8-hydroxynaphthalin-3,5-disulfonsäure, 2-Amino-5-hydroxynaphthalin-7,1-disulfonsäure und 2-Amino-8-hydroxynaphthalin-3,6-disulfonsäure.

Besonders geeignet ist das erfindunsgemäße Verfahren für die Umsetzung von Cyanurfluorid mit 2-Amino-5-hydroxynaphthalin-7-sulfonsäure, 2-Amino-8-hydroxynaphthalin-6-sulfonsäure, 1-Amino-8-hydroxynaphthalin-4,6-disulfonsäure oder vor allem 1-Amino-8-hydroxynaphthalin-3,6-disulfonsäure oder 1-Aminobenzol-3-sulfonsäure.

Die sulfogruppenhaltigen oder chromophorhaltigen Amine werden als wäßrige Lösungen eingesetzt. Man kann das Cyanurfluorid in größerem Überschuß verwenden, Zweckmäßig ist es, Cyanurfluorid und Amin im Molverhältnis 0,8:1 bis 1,5:1, vorzugsweise 1:1 bis 1,2:1, insbesondere 1:1 bis 1,08:1 einzusetzen.

Vorzugsweise setzt man der Aminlösung eine Puffersubstanz zu, die bewirkt, daß sich während der Umsetzung, je nach Puffersubstanz, ein pH-Wert zwischen 1 und 8, vorzugsweise zwischen 3 und 5 einstellt, Geeignete Puffersubstanzen sind z.B. Alkalimetallfluoride, -borate und -phosphate, insbesondere NaF, Diese Puffersubstanzen werden im allgemeinen in einer Menge von 0,2 bis 2, vorzugsweise 0,4 bis 1,2 Mol je Mol Amin eingesetzt.

Die erhaltenen Reaktionsprodukte aus 1 Mol Amin und 1 Mol Cyanurfluorid können isoliert werden, sie werden jedoch vorzugsweise ohne Zwischenisolierung weiterverarbeitet, z.B. zu Reaktivfarbstoffen durch Umsetzung mit einem weiteren Amin, aminogruppenhaltigen Farbstoff oder durch Umsetzung mit einem aromatischen Amin und nachfolgende Kupplung mit einer Diazoniumverbindung. Diese Weiterverarbeitung kann diskontinuierlich oder kontinuierlich auf bekannte Art und Weise durchgeführt werden.

Nach dem erfindungsgemäßen Verfahren erhält man die Kondensationsprodukte aus Cyanurfluorid und sulfogruppenhaltigen oder chromophorhaltigen aromatischen Aminen in vielen Fällen in deutlich höherer Reinheit als nach den bisher üblichen Verfahren. Dies wirkt sich positiv auf die Qualität der aus den Kondensationsprodukten hergestellten Reaktivfarbstoffen aus, da üblicherweise die Kondensationsprodukte ohne Zwischenreinigung weiterverarbeitet werden.

Das neue Verfahren läßt sich in unterschiedlichen Reaktoren durchführen, soweit sie eine intensive Durchmischung, eine rückvermischungsfreie Durchströmung und eine kontinuierliche Arbeitsweise erlauben.

Als besonders geeignet für die Durchführung des neuen Verfahrens hat sich ein Rohr als Reaktor erwiesen, in das zur Einführung der Edukte eine Düse axial und eine oder mehrere Düsen konzentrisch dazu einmünden.

Gemäß einer besonderen Ausführungsform ist die Austrittsrichtung der konzentrisch einmündenden Düsen(n) 45 bis 90° zur Austrittsrichtung der axialen Düse geneigt. Hierdurch findet ein besonders intensiver Stoffaustausch zwischen den beiden Eduktströmen statt.

Das Rohr setzt sich in Strömungsrichtung fort und dient hinter der Reaktionszone der Abführung der Reaktionsprodukte, dukte, wobei unmittelbar die an den Einmündungen beginnende Reaktionszone nur eine kurze Distanz ausmacht. Für das Einbringen der Reaktionspartner eignet sich besonders gut eine Zweistoffdüse oder eine zentrale Düse und eine diese umgebenden Ringspalt, wobei das Cyanurfluorid zentral eingeführt wird und dementsprechend die wäßrige Aminlösung konzentrisch dazu.

Der bevorzugt verwendete Reaktor ist in der Zeichnung rein schematisch dargestellt und nachstehend näher erläutert: Es zeigen:
- Fig. 1: den Reaktor im Schnitt und
- Fig. 2: die Düse gemäß Einzelheit A, zur Hälfte im Schnitt in vergrößerter Darstellung.

Ein Reaktor dieses Typs jedoch für Gas-Flüssig-Reaktionen ist aus Ullmanns Enclylopädie der technischen Chemie, Verlag Chemie, Weinheim, Bergstr., 4. Auflage (1973), Seiten 379-385 bekannt.

In ein Reaktionsrohr 1 von F_{R} = 80 mm² Kreisquerschnitt mündet axial über eine Düse 2 von F₁ = 0,03 mm² Querschnitt eine Zuleitung 3 für 2,4,6-Trifluor-s-triazin (Cyanurfluorid). Sie ist konzentrisch von einer Ringdüse 4 von F₂ = 64 mm² Querschnitt umgeben, welche mit einer Zuleitung 5 für eine wäßrige Aminlösung verbunden ist. Die Länge der Reaktionszone L ergibt sich aus der Verweilzeit von 5 s, vorzugsweise 2 s und der Geschwindigkeit der Gesamtströmung von Cyanurfluorid und Aminlösung. Der anschließende Rohrabschnitt 6 dient als Transportleitung für das Reaktionsprodukt.

Anstelle einer Ringdüse 4 können auch mehrere einzelne Düsen bzw. Düsenöffnungen über den Umfang verteilt sein.

### Beispiel 1

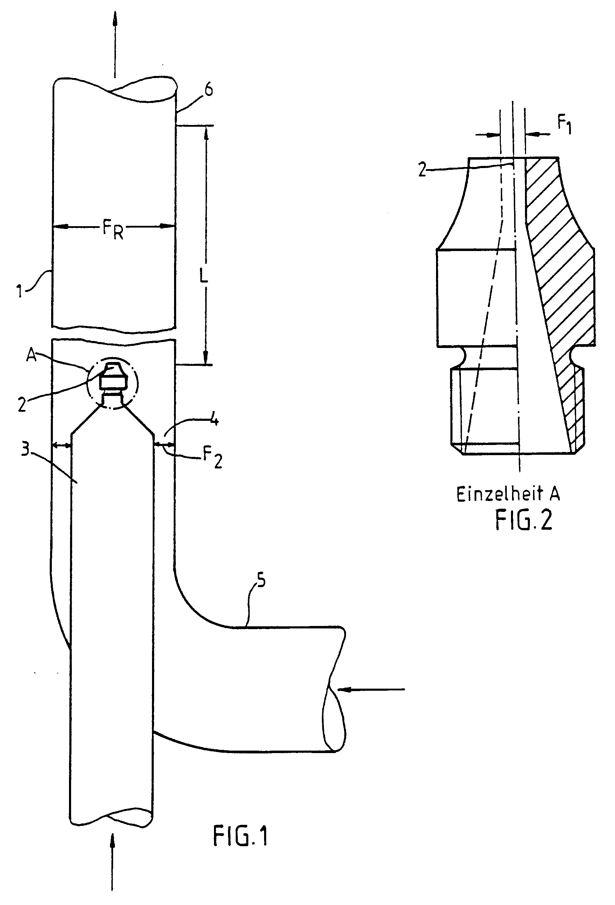

In einem Reaktor gemäß Fig.1 werden gleichzeitig und kontinuierlich über getrennte Zuleitungen 3 und 5 bzw. Düsenöffnungen 2 und 4 5,4 l/h Cyanurfluorid mit einer Temperatur von ca. 20°C und einer Reynolds-Zahl von 16.000 und 225 l/h einer wäßrigen Lösung von 0°C, enthaltend 0,26 Mol/l 1-Amino-8-hydroxynaphthalin-3,6-disulfonsäure, 0,1 Mol/l NaF und 0,26 Mol/l NaOH mit einer Reynolds-Zahl von 8.000 eingeleitet. Das Strömungsquerschnittsverhältnis zwischen dem Querschnitt F_{R} des Reaktors 2 und dem Querschnitt F₁ der Düse 2 beträgt 2.660.

Dabei wird das Cyanurfluorid mit einer Strömungsgeschwindigkeitsdifferenz von 49 m/s in die wäßrige Lösung eingedüst. Die Verweilzeit in der Reaktionszone beträgt etwa 0,7 s, d.h. in dieser Zeit ist die Reaktion im wesentlichen abgeschlossen.

Das Reaktionsgemisch, das die Reaktionszone L (L = 570 mm) mit einer Temperatur von 9°C verläßt, enthält die Verbindung der Formel 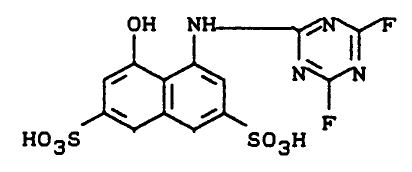
in einer Ausbeute von 93 ± 2 %, bezogen auf die eingesetzte 1-Amino-8-hydroxynaphthalin-3,6-disulfonsäure.

Vergleichsweise werden in dieser Reaktionsstufe mit dem Verfahren gemäß EP-A-172 790 (Seite 6 oben) nur 85 % erreicht.

Die erhaltene Reaktionsmischung wird in einen Rührkessel geleitet und dort nach der folgenden Vorschrift in einen roten Reaktivfarbstoff überführt:

Zu der Reaktionsmischung gibt man pro Mol des vorstehend beschriebenen Kondensationsproduktes eine Lösung, enthaltend 1 Mol p-Chloranilin, 500 ml Wasser und 100 ml Salzsäure von 32 % und stellt innerhalb von 10 bis 20 Minuten mit 20 %iger NaOH einen pH-Wert von 6,0 ein.

Die erhaltene Lösung gibt man zu einer Diazoniumsalz-Suspension von 0 bis 5°C, welche durch Diazotierung auf übliche Weise von 1 Mol 2-Naphthylamin-1,5-disulfonsäure erhalten wurde, Bei max, 10°C stellt man unter intensivem Rühren mit 30 %iger Natronlauge einen pH-Wert von 7,5 ein und rührt noch 1 Stunde bei pH 7,5 und 10°C.

Man erhält den Farbstoff der Formel 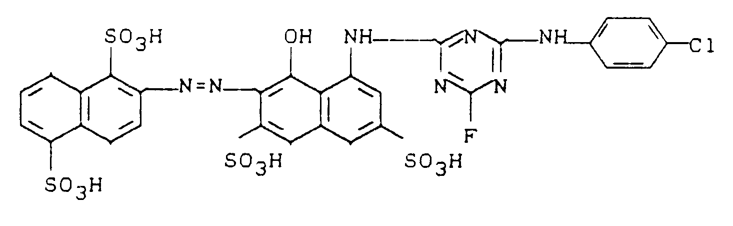
der Cellulosematerial in roten Tönen färbt.

### Beispiel 2

In einen Reaktor 1 gemäß Fig. 1 werden gleichzeitig und kontinuierlich über getrennte Zuleitungen 3 und 5 bzw. Düsenöffnungen 2 und 4 5,4 l/h Cyanurfluorid mit einer Temperatur von ca. 20°C und einer Reynolds-Zahl von 16.000 und 225 l/h einer wäßrigen Lösung von 0°C, enthaltend 0,26 Mol/l 1-Amino-8-hydroxynaphthalin-3,6-disulfonsäure, 0,1 Mol/l NaF und 0,26 Mol/l Morpholin mit einer Reynolds-Zahl von 8.000 eingeleitet. Das Strömungsquerschnittverhältnis zwischen dem Querschnitt F_{R} des Reaktors 1 und dem Querschnitt F₁ der Düse 2 beträgt 2.660.

Dabei wird das Cyanurfluorid mit einer Differenzströmungsgeschwindigkeit von 49 m/s in die wäßrige Lösung eingedüst. Die Verweilzeit im Reaktor 1 beträgt 0,7 s, was einer Länge L von 570 mm entspricht.

Das Reaktionsgemisch, das den Reaktor mit einer Temperatur von 9°C verläßt, enthält die Verbindung der Formel 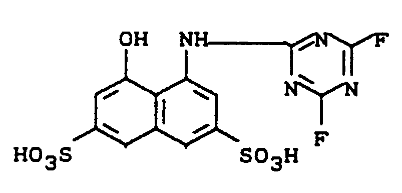
in Form des Morpholinsalzes in einer Ausbeute von 93 ± 2 %.

Durch Einstellen des pH-Wertes von 7 mit LiOH wird die Kupplungskomponente der Formel 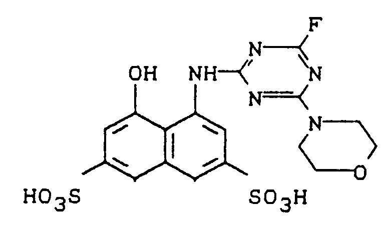
erhalten, die analog Beispiel 1 mit einer entsprechenden Diazotierung zum Farbstoff der Formel 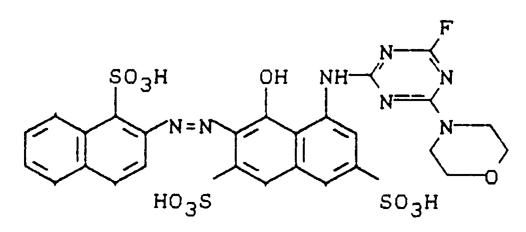
weiterverarbeitet wird. Der Farbstoff färbt Cellulose in blaustichig roten Tönen mit sehr hoher Naßechtheit.

## Patentansprüche

1. Verfahren zur kontinuierlichen Umsetzung von Cyanurfluoriden mit Aminen, wobei Cyanurfluorid und eine wäßrige Aminlösung in einen Reaktor (1) eingeleitet werden und das Reaktionsprodukt anschließend abgeleitet wird, dadurch gekennzeichnet, daß die Edukte gleichzeitig und kontinuierlich mit unterschiedlichen Geschwindigkeiten in den Reaktor (1) eingeleitet werden, daß durch die Differenz der Strömungsgeschwindigkeiten eine intensive Vermischung bewirkt wird, und daß die Reaktion bei rückvermischungsfreier Strömung im wesentlichen in diesem Reaktor (1) abgeschlossen wird.

2. Verfahren nach Anspruch 1, dadurch gekennzeichnet, daß das Cyanurfluorid mit einer Reynolds-Zahl von mindestens 10.000 und die wäßrige Aminlösung mit einer solchen von mindestens 2.500 in den Reaktor (1) einströmen, wobei die Differenz der Strömungsgeschwindigkeiten zwischen Cyanurfluoridstrom und Aminlösungsstrom mindestens 20 m/s, vorzugsweise mindestens 40 m/s beträgt.

3. Verfahren nach Anspruch 1, dadurch gekennzeichnet, daß ein Verhältnis des Reaktorquerschnittes F_{R} zum Einströmquerschnitt F₁ des Cyanurfluoridstromes von 225 bis 40.000, vorzugsweise von 700 bis 12.000, eingehalten wird.

4. Verfahren nach Anspruch 1, 2 oder 3, dadurch gekennzeichnet, daß die Verweilzeit im Reaktor (1) maximal 5 s beträgt.

5. Verfahren nach einem der Ansprüche 1 bis 4, dadurch gekennzeichnet, daß die Reaktion bei Temperaturen von 0 bis 50°C durchgeführt wird

6. Verfahren nach einem der Ansprüche 1 bis 5, dadurch gekennzeichnet, daß 2,4,6-Trifluor-s-triazin mit sulfogruppenhaltigen Anilinen und Naphthylaminen oder mit sulfogruppenhaltigen Aminonaphtholen umgesetzt wird.

7. Verfahren nach einem der Ansprüche 1 bis 5, dadurch gekennzeichnet, daß 2,4,6-Trifluor-s-triazin mit chromophorhaltigen Aminen umgesetzt wird.

8. Verfahren nach Anspruch 1, in dem der Reaktor (1) ein Rohr ist, in das zur Einführung eine Düse (2) axial und eine oder mehrere Düsen (4) konzentrisch dazu einmünden,

9. Verfahren nach Anspruch 8, dadurch gekennzeichnet, daß die Austrittsrichtung der konzentrisch einmündenden Düse(n) (4) 45 bis 90° zur Austrittsrichtung der axialen Düse (2) geneigt ist.

10. Verfahren nach Anspruch 8 oder 9, dadurch gekennzeichnet, daß das Verhältnis des Reaktorquerschnittes F_{R} zum Einströmquerschnitt F₁ des Cyanurfluoridstromes zwischen 400 und 12.000 beträgt.

## Claims

1. Process for the continuous reaction of cyanuric fluorides with amines, with cyanuric fluoride and an aqueous amine solution being fed into a reactor (1) and the reaction product subsequently being transferred out, characterized in that the starting materials are simultaneously and continuously fed into the reactor (1) at different velocities, that the difference in the flow velocities effects intensive mixing, and that the reaction is essentially concluded in this reactor (1) with backmixing-free flow.

2. Process according to Claim 1, characterized in that the cyanuric fluoride flows into the reactor at a Reynolds number of at least 10,000 and the aqueous one solution flows into the reactor (1) at a Reynolds number of at least 2,500, with the difference in the flow velocities between the cyanuric fluoride stream and the amine solution stream being at least 20 m/s, preferably at least 40 m/s.

3. Process according to Claim 1, characterized in that the ratio of the reactor cross-section F_{R} to the inflow cross-section F₁ of the cyanuric fluoride stream is kept at from 225 to 40,000, preferably from 700 to 12,000.

4. Process according to Claim 1, 2 or 3, characterized in that the residence time in the reactor (1) is at most 5 s.

5. Process according to one of Claims 1 to 4, characterized in that the reaction is carried out at temperatures of from 0 to 50°C.

6. Process according to one of Claims 1 to 5, characterized in that 2,4,6-trifluoro-s-triazine is reacted with anilines and naphthylamines containing sulpho groups or with aminonaphthols containing sulpho groups.

7. Process according to one of Claims 1 to 5, characterized in that 2,4,6-trifluoro-s-triazine is reacted with amines containing chromophores.

8. Process according to Claim 1, in which the reactor (1) is a tube into which enter, for introducing material, a nozzle (2) in an axial direction and one or more nozzles (4) concentric thereto.

9. Process according to Claim 8, characterized in that the discharge direction of the concentrically entering nozzle(s) (4) is inclined at from 45 to 90° to the discharge direction of the axial nozzle (2).

10. Process according to Claim 8 or 9, characterized in that the ratio of the reactor cross-section F_{R} to the inflow cross-section F₁ of the cyanuric fluoride stream is between 400 and 12,000.

## Revendications

1. Procédé pour la réaction en continu de fluorures cyanuriques avec des amines, le fluorure cyanurique et une solution aqueuse d'amine étant introduits dans un réacteur (1) et le produit de réaction état ensuite dévié, caractérisé en ce qu'on introduit dans le réacteur (1) les éduits simultanément et en continu avec des vitesses différentes, qu'on provoque a mélange intensif par l'intermédiaire de la différence des vitesses d'écoulement et que la réaction est achevée dans l'essentiel dans ce réacteur (1) en présence d'un écoulement exempt de remélangeage.

2. Procédé selon la revendication 1, caractérisé en ce que le fluorure cyanurique entre avec un nombre de Reynolds d'au moins 10 000 et que la solution aqueuse d'amine entre avec un tel nombre d'au moins 2500 dans le réacteur (1), la différence des vitesses d'écoulement entre le courant de fluorure cyanurique et le courant de la solution d'amine étant au moins de 20 m/s, de préférence au moins de 40 m/s.

3. Procédé selon la revendication 1, caractérisé en ce qu'on respecte un rapport de la section transversale du réacteur F_{R} à la section transversale d'entrée F₁ du courant de fluorure cyanurique de 225 à 40 000, de préférence de 700 à 12 000.

4. Procédé selon la revendication 1, 2 ou 3, caractérisé en ce que le temps de séjour dans le réacteur (1) s'élève au maximum à 5 s.

5. Procédé selon l'une quelconque des revendications 1 à 4, caractérisé en ce qu'on effectue la réaction à des températures de 0 à 50°C.

6. Procédé selon l'une quelconque des revendications 1 à 5, caractérisé en ce qu'on fait réagir la 2,4,6-trifluoro-s-triazine avec des anilines et des naphtylamines contenant des groupes sulfonés ou avec des aminonaphtols contenant des groupes sulfonés.

7. Procédé selon l'une quelconque des revendications 1 à 5, caractérisé en ce qu'on fait réagir la 2,4,6-trifluoro-s-triazine avec des amines contenant des chromophores.

8. Procédé selon la revendication 1, dans lequel le réacteur (1) est un tuyau, dans lequel débouchent pour l'introduction une buse (2) de manière axiale et en sus une ou plusieurs buses (4) de manière concentrique.

9. Procédé selon la revendication 8, caractérisé en ce que la direction de sortie de la (des) buse(s) (4) débouchant de manière concentrique est inclinée de 45 à 90° par rapport à la direction de sortie de la buse axiale (2).

10. Procédé selon la revendication 8 ou 9, caractérisé en ce que le rapport de la section transversale du réacteur F_{R} à la section transversale d'entrée F₁ du courant de fluorure cyanurique est comprise entre 400 et 12 000.
